# EUROPEAN PATENT APPLICATION

(11) **EP 3 431 068 A1**
(43) Date of publication of application: **23.01.2019**
(21) Application number: 17181952.7
(22) Date of filing: 18.07.2017
(51) Int. Cl.: A61H 3/00, A47C 9/02, A61F 5/01

(54) **WEARABLE SITTING POSTURE ASSISTING DEVICE AND METHOD FOR USING A WEARABLE SITTING POSTURE ASSISTING DEVICE**

(71) Applicant: noonee AG, 8630 Rüti ZH (CH)
(72) Inventor: GUNURA, Keith, 8003 Zürich (CH); VAFI, Daniel, 8006 Zürich (CH); MOTOVILOVA, Olga, 8045 Zürich (CH)
(74) Representative: Daub, Thomas

(57) **Abstract**

The invention relates to a wearable sitting posture assisting device, comprising a functional unit (10a) which is configured for implementing at least one posture function, in particular a posture assisting function, for a person (200a) in at least one wearing state.

It is proposed that the wearable sitting posture assisting device (10a) comprises at least one safety unit (12a-c) which, in at least one disabling state, at least partly disables the posture function and which, in at least one enabling state, at least partly enables the posture function.

## Description

### State of the Art

The invention relates to a wearable sitting posture assisting device according to the preamble of claim 1 and to a method for using a wearable sitting posture assisting device according to the preamble of claim 15.

A posture assisting device is known from document WO 2015/028373 A1.

An objective of the invention is, in particular, to provide a generic wearable sitting posture assisting device featuring improved characteristics regarding safety. Another objective of the invention is, in particular, to prevent injuries and/or accidents due to user errors. Yet another objective of the invention is, in particular, to improve safety and/or comfort when several people are sharing a wearable sitting posture assisting device. The objective is achieved according to the invention by the features of patent claims1 and 15, while advantageous embodiments and further developments of the invention may be gathered from the dependent claims.

### Advantages of the Invention

The invention relates to a wearable sitting posture assisting device, comprising a functional unit which is configured for implementing at least one posture function, in particular a posture assisting function, for a person in at least one wearing state.

It is proposed that the wearable sitting posture assisting device comprises at least one safety unit, which at least partly disables the posture function, in particular the posture assisting function, in at least one disabling state, and which at least partly enables the posture function, in particular the posture assisting function, in at least one enabling state.

By means of the invention a wearable sitting posture assisting device can be provided featuring improved characteristics regarding safety. In particular, accidents and/or injuries due to user errors can be prevented. Furthermore, safety can be improved by inducing a user to check the condition of a wearable sitting posture assisting device prior to using it. Advantageously, proper adjustment of adjustable components can be ensured, in particular if several people are sharing a wearable sitting posture assisting device. In addition, low costs due to reduced maintenance effort can be achieved. Furthermore, weakened components and/or imminent defects can be identified ahead of time.

A "wearable sitting posture assisting device" is herein to be understood as a device which is configured for receiving a weight force of a person in a sitting posture or in a partly sitting posture and in particular for transmitting the weight force to a ground. In particular, an angle between a thigh and a shank of at least one leg of the person in the sitting posture is no greater than 130°, preferably no greater than 120° and advantageously no greater than 110°, and/or no less than 60°, preferably no less than 70° and advantageously no less than 80°. In particular, the angle between the thigh and the shank of the person is approximately 90° in the sitting posture. In particular, an angle between a thigh and a shank of the person in the partly sitting posture is no greater than 170°, preferably no greater than 160° and advantageously no greater than 150°, and/or no less than 100°, preferably no less than 110° and advantageously no less than 120°. In particular, the angle between the thigh and the shank of the person is approximately 130° in the partly sitting posture. It is conceivable that the partly sitting posture is a posture in which the person leans forward while partly bending his knees. In particular, a person wearing the wearable sitting posture assisting device is enabled to sit and/or to partly sit and/or to sit down on the wearable sitting posture assisting device, wherein at least a portion of the weight force is counteracted by the wearable sitting posture assisting device, and/or wherein the person counteracts only a fraction of the weight force using his muscles. In particular, the wearable sitting posture assisting device is configured for being worn by the person while the person is standing and/or while the person is walking. Advantageously, the wearable sitting posture assisting device is configured for supporting different sitting postures and/or partly sitting postures, which are in particular characterized by different sitting angles.

The wearable sitting posture assisting device in particular defines a sitting direction. Preferably, the person faces and/or looks in the sitting direction when sitting or partly sitting on the wearable sitting posture assisting device and facing forward. In particular, the sitting direction is oriented parallel to a floor on which the person is standing and/or sitting and/or walking when wearing the wearable sitting posture assisting device. In particular, the wearable sitting posture assisting device defines a walking direction. Preferably, the person faces in the walking direction when walking and/or standing with the wearable sitting posture assisting device and facing forward. In particular, the walking direction is oriented parallel to a floor on which the person is standing and/or sitting and/or walking when wearing the wearable sitting posture assisting device. In particular, the wearable sitting posture assisting device is only designed to receive and transmit the weight force. Preferably, the wearable sitting posture assisting device is not designed to generate a controllable force configured to assist a person while walking, standing or lifting loads. In particular, the wearable sitting posture assisting device is a mechanical wearable sitting posture assisting device and preferably free of a drive unit, in particular free of an electric drive and/or free of an engine. "Configured" is in particular to mean specifically programmed, designed and/or equipped. By an object being configured for a certain function is in particular to be understood that the object implements and/or fulfills said certain function in at least one application state and/or operating state.

Preferably, the wearable sitting posture assisting device comprises at least one leg unit. In particular, the wearable sitting posture assisting device comprises at least one additional leg unit. Preferably, the functional unit comprises the leg unit. Furthermore, the functional unit in particular comprises the additional leg unit. In particular, the leg unit comprises at least one, preferably one, upper leg and/or at least one, preferably one, lower leg and/or comprises at least one, preferably one, foot unit, and/or comprises at least one, preferably one, ground contact unit. Advantageously, the upper leg is connected to the lower leg, in particular via at least one, in particular one, knee joint. Preferably, the foot unit is connected to the lower leg. Advantageously, the ground contact unit is connected to the lower leg and/or to the foot unit. It is conceivable that the ground contact unit is at least partly implemented integrally with the lower leg and/or at least partly implemented integrally with the foot unit. It is also conceivable that the foot unit is at least partly implemented integrally with the lower leg. Preferably, the wearable sitting posture assisting device comprises at least one upper body wearing unit. In particular, the leg unit is connected to the upper body wearing unit, preferably via at least one connection strap. In this context, the term "a first object and a second object being at least partly implemented integrally" is in particular to mean that at least one component of the first object and at least one component of the second object are implemented integrally with each other. "Implemented integrally" is in particular to mean, in this context, connected at least by substance-to-substance bond, e.g., by a welding process, an adhesive bonding, an injection-molding process and/or by another process that is deemed expedient by a person having ordinary skill in the art. Advantageously, "implemented integrally" could in particular mean made of one piece. "Made of one piece" is, in particular, to mean, in this context, manufactured from one single piece, e.g., by production from one single cast and/or by manufacturing in a one-component or multi-component injection-molding process, and advantageously from a single blank.

Preferably, the wearable sitting posture assisting device comprises two leg units. Advantageously, the leg unit and the additional leg unit are implemented identically. It is also conceivable that the leg unit and the additional leg unit are implemented mirror-symmetrically with respect to each other. It is conceivable that the leg unit is configured for being worn on a left leg and the additional leg unit is configured for being worn at a right leg, or vice versa. Advantageously, the leg unit is configured for being worn either on a left leg or on a right leg. Further advantageously, the additional leg unit is configured for being worn on a left leg or on a right leg. Preferably, the additional leg unit is connected to the upper body wearing unit, preferably via at least one connection strap. In particular, the person wearing the wearable sitting posture assisting device wears the leg unit, in particular solely, on a first leg, for instance a left leg or a right leg. In particular, the person wearing the wearable sitting posture assisting device wears the additional leg unit, in particular solely, on a second leg, for instance a right leg or a left leg. Advantageously, the leg unit is arranged on a rear side of the leg on which the leg unit is worn. Further advantageously, the additional leg unit is arranged on a rear side of the leg on which the additional leg unit is worn. In particular, the leg units of the wearable sitting posture assisting device are arranged on respective rear sides of the legs of the person when the person is sitting and/or partly sitting and/or sitting down on the wearable sitting posture assisting device and/or standing and/or walking with the wearable sitting posture assisting device. Preferably, the person wearing the wearable sitting posture assisting device wears the upper body wearing unit on his upper body. Advantageously, the upper body wearing unit is implemented as a belt and/or as braces and/or as suspenders. The wearable sitting posture assisting device enables the person wearing it to walk around, to stand, to sit down on the wearable sitting posture assisting device if required or desired and to stand up after sitting or partly sitting on the wearable sitting posture assisting device.

Preferably, the wearable sitting posture assisting device comprises at least one thigh connection unit for connecting the leg unit, in particular the upper leg, to a thigh of the person. Preferably, the thigh connection unit features at least one thigh strap. In particular, the wearable sitting posture assisting device comprises a seat unit configured for providing at least one sitting surface for the person, in particular if the person is sitting or partly sitting on the wearable sitting posture assisting device, preferably for the thigh and/or for at least a lower portion of a buttock of the person, wherein "buttock" is preferably to mean one cheek of the buttocks. Advantageously, the seat unit is connected to and/or connectable to the upper leg. Preferably, the seat unit comprises at least one sitting element which features the sitting surface. Advantageously, the seat unit is in contact with the thigh of the person if the person is sitting or partly sitting on the wearable sitting posture assisting device. Preferably, the seat unit is arranged on a rear side of the thigh of the person if the person is standing or walking with the wearable sitting posture assisting device.

Advantageously, the upper leg comprises at least one upper leg support. Preferably, the seat unit is connected to and/or connectable to the upper leg support. Advantageously, the thigh connection unit and/or the thigh strap is connected to and/or connectable to the upper leg support. In particular, the upper leg support is implemented as a frame element. Preferably, the upper leg support is implemented as an elongate element. Advantageously, the upper leg features at least one upper leg longitudinal axis which is oriented at least substantially parallel to a longitudinal axis of the thigh of the person. Preferably, a main extension direction of the upper leg support is oriented at least substantially parallel, or parallel, to the upper leg longitudinal axis. In particular, the upper leg support is at least partly, preferably at least to a large extent, advantageously completely made of plastic. It is also conceivable that the upper leg support is at least partly, preferably at least to a large extent, advantageously completely made of metal, in particular made of a light metal or a light alloy, for instance aluminum and/or titanium and/or beryllium and/or scandium or another suitable metal. It is further conceivable that the upper leg support is at least partly, preferably at least to a large extent, advantageously completely made of a composite material, in particular a fiber reinforced composite material and/or a fiber reinforced plastic and/or a carbon fiber reinforced material and/or a carbon fiber reinforced polymer and/or a fiber reinforced thermoplastic. The term "at least to a large extent" is in particular to mean to an extent of at least 55 %, preferably to an extent of at least 65 %, further preferably to an extent of at least 75 %, advantageously to an extent of at least 85 % and further advantageously to an extent of at least 95 %. In this context "at least substantially parallel" is in particular to mean an orientation of a direction with respect to a reference direction, in particular in a plane, wherein the direction differs from the reference direction in particular by less than 15º, advantageously by less than 10º and particularly advantageously by less than 2º. A "main extension direction" of an object is, in particular, to be understood, in this context, as a direction extending in parallel to a largest side of an imaginary rectangular cuboid which only just entirely encloses the object.

Preferably, the lower leg is arranged on a rear side of the shank of the person in at least one wearing state. In particular, the lower leg comprises at least one lower leg support. Advantageously, the lower leg support is implemented as a frame element. Preferably, the lower leg support is implemented as an elongate element. In particular, the lower leg features at least one lower leg longitudinal axis, which is oriented at least substantially parallel to a longitudinal axis of the shank of the person. Preferably, a main extension direction of the lower leg support is oriented at least substantially parallel, or parallel, to the lower leg longitudinal axis. Advantageously, the lower leg longitudinal axis is oriented at least substantially parallel, or parallel, to the upper leg longitudinal axis.

Preferably, the upper leg and the lower leg together define a sitting angle. Advantageously, the sitting angle is an angle included by the upper leg longitudinal axis and the lower leg longitudinal axis, in particular on a rear side of the upper leg and the lower leg. In particular, in the sitting posture the sitting angle is no greater than 130°, preferably no greater than 120° and advantageously no greater than 110°, and/or no less than 60°, preferably no less than 70° and advantageously no less than 80°. In particular, the sitting angle is approximately 90° in the sitting posture. In particular, in the partly sitting posture the sitting angle is no greater than 170°, preferably no greater than 160° and advantageously no greater than 150°, and/or no less than 100°, preferably no less than 110° and advantageously no less than 120°. In particular, the sitting angle is approximately 130° in the partly sitting posture. Preferably, the sitting angle at least approximately equals the angle between the thigh and the shank of the person. In particular, the sitting angle is approximately 180° in a standing posture.

In particular, the knee joint pivotably connects the lower leg to the upper leg, preferably pivotably about a knee joint axis. Advantageously, the knee joint axis is oriented at least substantially perpendicularly, or perpendicularly, to the upper leg longitudinal axis and/or to the lower leg longitudinal axis. Preferably, the knee joint axis is oriented at least substantially perpendicularly, or perpendicularly, to the sitting direction. Advantageously, the upper leg support and the lower leg support together implement at least a portion of the knee joint, or the knee joint. Preferably, a value of the sitting angle corresponds to a value of a knee joint position of the knee joint. In this context "at least substantially perpendicularly" is in particular to mean an orientation of a direction with respect to a reference direction, in particular in a plane, wherein the direction and the reference direction include an angle which differs from an angle of 90° by no more than 15º, advantageously by no more than 10º and particularly advantageously by no more than 2º.

Preferably, the wearable sitting posture assisting device comprises at least one locking unit, which is configured for locking the upper leg with respect to the lower leg and/or to the knee joint in a certain sitting angle and/or is configured for defining a smallest sitting angle. Advantageously, the locking unit is configured for locking the knee joint in different sitting angles, and/or for defining different smallest sitting angles, which sitting angles can be preferably chosen by the person. It is conceivable that the locking unit is configured for allowing, in a locked state, the sitting angle to be increased. In particular, the person is enabled to stand up when the locking unit is in the locked state. Preferably, the locking unit is configured for enabling the person to sit down again at the defined smallest sitting angle after standing up, with the locking unit still defining the same smallest sitting angle. Advantageously, the locking unit comprises at least one blocking element, which is configured for blocking and/or unblocking the knee joint and/or for fixating the sitting angle and/or for defining a smallest sitting angle. Preferably, the blocking element is implemented as a spring, in particular as a gas spring. Advantageously, the blocking element is connected to the upper leg, in particular to the upper leg support, and to the lower leg, in particular to the lower leg support. Preferably, the blocking element is configured for damping a movement of the upper leg with respect to the lower leg during sitting down and/or during standing up. Advantageously, the locking unit features at least one actuation element, which is configured for actuating the blocking element. Preferably, the actuation element is configured for enabling the person to block or unblock the blocking element. Advantageously, the actuation element is a mechanical actuation element. It is also conceivable that the actuation element is an electronical actuation element. It is further conceivable that the locking unit comprises at least one control unit, which is configured for detecting a sitting-down condition when the person sits down and/or detecting a standing-up condition when the person stands up and/or triggering the actuation element according to a requirement of blocking the blocking element.

In particular, the wearable sitting posture assisting device comprises a foot unit, which foot unit may be configured for connecting to a foot and/or shoe of the person and/or which foot unit may be connected to a foot and/or shoe of the person. Preferably, the foot unit comprises at least one foot connector for connecting to the foot and/or shoe of the person. Advantageously, the foot connector features at least one shoe strap. In particular, the foot connector may feature at least one upper strap, which advantageously runs across an instep of the foot and/or shoe which the foot unit is connected to. Preferably, a foot connector may feature at least one lower strap, which advantageously runs across a sole of the foot and/or shoe which the foot unit is connected to. Preferably, a foot connector is configured for being worn on a foot and/or shoe. Advantageously, the foot unit comprises at least one foot unit support. Preferably, the foot connector is connected to the foot unit support. Advantageously, the foot unit support comprises at least one bracket, and/or the foot unit support is implemented as a bracket. Preferably, the shoe strap, in particular the upper strap and/or the lower strap, is connected to the bracket. It is conceivable that the foot unit is connected to and/or connectable to the lower leg, in particular to the lower leg support. It is further conceivable that the foot unit support element could be at least partly implemented integrally, or implemented integrally, with the lower leg, in particular with the lower leg support.

Additionally or alternatively, in particular alternatively to the foot unit, the wearable sitting posture assisting device, in particular the leg unit, may comprise at least one shank connection unit for connecting to the shank of the person. In particular, the wearable sitting posture assisting device may comprise the shank connection unit and be free of a foot unit and/or a foot connector. The wearable sitting posture assisting device may also comprise both at foot connector and a shank connector, in particular both assigned to the same leg unit.

In particular, the ground contact unit comprises at least one ground contact element. Preferably, the ground contact element features at least one ground contact surface, which is advantageously configured for contacting a ground when the person is sitting or partly sitting on the wearable sitting posture assisting device. In particular, the ground contact element is at least partly, preferably at least to a large extent, advantageously completely made of rubber. Preferably, a weight force of the person is transmitted from the seat unit to the upper leg support and/or from the upper leg support to the knee joint and/or from the knee joint to the lower leg support and/or from the lower leg support to the ground contact element and/or from the ground contact element to the ground. In particular, the weight of the person is additionally transmitted to the ground via the foot and/or shoe of the person. Preferably, the ground contact element is arranged on a rear side of the foot and/or shoe of the person. When the person is sitting or partly sitting on the wearable sitting posture assisting device, the foot and/or shoe of the person is in contact with the ground in addition to the ground contact element. Preferably, the ground contact element is arranged contactlessly with respect to the ground when the person walks or stands while wearing the sitting posture assisting device.

By a first object being connected to a second object is in particular to be understood that the first object and the second object are directly connected and/or that at least a portion of the first object directly mechanically contacts the second object. It is further conceivable that the first object is, in particular directly, fixated to the second object and/or vice versa. It is also conceivable that the first object and the second object are at least partly implemented integrally.

Preferably, the posture function is a posture assisting function. In particular, the posture function may encompass providing support for the person when the person is sitting or partly sitting on the wearable sitting posture assisting device. Furthermore, the posture function may comprise allowing the person to temporarily stand up after sitting or partly sitting and/or before sitting down on the wearable sitting posture assisting device again. It is further conceivable that the posture function is a walking function and/or a standing-up function and/or a sitting down function, which posture function preferably allows the person bending the knee joint and/or moving components of the leg unit by forces exerted onto the wearable sitting posture assisting device while walking and/or standing up and/or sitting down while wearing the wearable sitting posture assisting device. In particular, the posture function may encompass different functionalities provided by the wearable sitting posture assisting device which can be requested and/or used by the person wearing the wearable sitting posture assisting device consecutively and/or simultaneously. Preferably, the posture function is a load relief function, which is in particular implemented by the leg unit and the additional leg unit, the leg unit and the additional leg unit further preferably transmitting at least a portion of the weight force of the person to the ground, thus supporting a body, in particular joints, of the person. Further preferably, the posture function is a mechanical function and/or a passive function, in particular a passive function implemented without a drive unit and/or without actively moving components of the functional unit.

In particular, the disabling state is a state of the safety unit. Preferably, the leg unit is in an at least partly disabled state, in particular in a disabled state, when the safety unit is in the disabling state. In particular, the enabling state is a state of the safety unit. Preferably, the leg unit is in an at least partly enabled state, in particular in an enabled state, when the safety unit is in the disabling state. Further preferably, the enabled state of the leg unit is a ready-to-use state. Preferably, the safety unit comprises at least one safety element. In particular, the safety element may be configured as a disabling element and/or a locking element and/or a blocking element or the like. Further preferably, the safety unit, in particular the safety element, disables the posture function in the disabling state. Furthermore, the safety unit, in particular the safety element, preferably enables the posture function in the enabling state. For instance, the safety unit may be configured for prohibiting sitting on the wearable sitting posture assisting device. Additionally or alternatively it is conceivable that the safety unit prohibits standing up and/or putting on or off the wearable sitting posture assisting device. Preferably, the safety unit remains in the disabling state and/or in the enabling state without consuming power, in particular without being held in position by a drive unit and/or in a powerless state of the safety unit and/or of the functional unit and/or of the wearable sitting posture assisting device.

Preferably, the safety unit is a safety check unit. In particular, the safety unit is configured for enabling the posture function depending on at least one safety check parameter. Preferably, the safety check parameter is a parameter defining whether or not at least one safety check step has been performed, in particular by a user. In particular, the safety unit is configured for enabling the posture function only if at least one safety check step has been performed by the user, in particular with a positive outcome, which positive outcome preferably encompasses the user having confirmed safety, for instance of a certain component and/or function of the wearable sitting posture assisting device, in the safety check step. Further preferably, the safety unit is configured for disabling the posture function if the safety check step has not been performed by the user. If the safety check step yields a positive result, the user may enable the posture function. In particular, the safety check step may comprise at least one release and/or clearance provided by the user, for instance following a visual inspection, a function testing, a performance test or the like.

In an advantageous embodiment of the invention it is proposed that, in the disabling state, the safety unit, in particular the safety element, blocks at least one movement, in particular a movement of at least one element of the functional unit with respect to at least one other element of the functional unit. In particular, the safety unit blocks the movement mechanically. Preferably, the safety element is configured for blocking the movement counter to a force, in particular a sitting force, which is for instance exerted onto the functional unit and/or the leg unit by the person attempting to sit on the wearable sitting posture assisting device, and/or a pulling force, for instance exerted onto the functional unit and/or the leg unit by the person attempting to stand up while wearing the wearable sitting posture assisting device, of more than 100 N, preferably of more than 200 N, further preferably of more than 500 N and advantageously of more than 1 kN. As a result, usage of the wearable sitting posture assisting device can be prevented by the safety unit in order to achieve a high degree of safety.

In a further embodiment of the invention it is proposed that the safety unit is configured for enabling the posture function depending on at least one user input. For instance, the user input may encompass at least one mechanical input, at least one electronical input, at least one acoustical input or the like. It is conceivable that the safety unit comprises at least one input unit, which is configured for inputting the user input. For instance, the input unit may comprise at least one button and/or at least one lever and/or at least one display, in particular a touch display, and/or at least one touch-sensitive surface and/or the like. Additionally or alternatively the safety unit may comprise at least one detection unit for detecting at least one user parameter, preferably the user input. For instance, the detection unit may comprise at least one optical and/or acoustical sensor, in particular a camera and/or a microphone and/or a fingerprint scanner and/or a retina recognition sensor and/or an iris recognition sensor or the like. It is conceivable that the detection unit is configured for identifying the user, for instance if the user attempts to release and/or clear a function after the safety check step, wherein in particular only users registered with the wearable sitting posture assisting device may have the authorization to do so. It is further conceivable that the safety unit comprises at least one network interface, which is configured for connecting to at least one data base, which data base may for instance comprise user data sets featuring at least one authorization information of the respective user, at least one safety requirement information, at least one usage parameter, at least one individualization parameter or the like. Preferably, the user input encompasses at least one mechanical actuation, in particular a mechanical actuation of at least one element of the safety unit, by the user. As a result, a user is enabled and/or induced to check the safety of a wearable sitting posture assisting device prior to wearing it. Furthermore, a high degree of safety can be achieved and/or maintenance requirements can be reliably identified since a user has to check the condition of the wearable sitting posture assisting device prior to wearing it.

In a further embodiment of the invention it is proposed that the safety unit comprises at least one disabling element, which is, in the disabling state, in at least one disabling position and which is, in the enabling state, in at least one enabling position. In particular, the disabling element disables the posture function in the disabling state. Preferably, the disabling element is the safety element and/or implements the safety element. Furthermore, the disabling element in particular enables the posture function in the enabling state and/or at least does not disable the posture function in the enabling state. Preferably, the disabling element is configured to be actuated by the user. Further preferably, the user input encompasses moving and/or switching and/or turning and/or bringing the disabling element from the disabling position into the enabling position. For instance, the disabling element may comprise and/or may be implemented as at least one blocking element, at least one key cylinder, at least one locking element, at least one combination lock, at least one padlock or the like. It is also conceivable that the safety unit comprises at least one actuator, which may be configured for moving and/or switching and/or turning and/or bringing the disabling element from the disabling position into the enabling position, in particular depending on the user input. As a result, usage of a wearable sitting posture assisting device in an unsafe condition can be advantageously prevented.

In yet a further embodiment of the invention it is proposed that the disabling position and/or the enabling position is a pushed-in position of the disabling element. In particular, the disabling position may be a pushed-in position and the enabling position may be a pushed-out position or vice versa. Preferably, the disabling element comprises at least one push button and/or is implemented as such, wherein the user input in particular comprises at least one pushing in of the disabling element. Further preferably, the disabling element and/or the push button is bi-stable. In particular, pushing the disabling element and/or the push button once may switch it from the disabling position into the enabling position, whereas pushing the disabling element and/or the push button for a second time may switch it from the enabling state back into the disabling state. Preferably, the disabling element is configured for latching and/or snapping and/or clicking into place in the disabling position and/or in the enabling position. As a result, operating errors during enabling of the posture function can be advantageously avoided. Furthermore, a safety check can be performed in a time-efficient and/or comfortable way.

In an advantageous embodiment of the invention it is proposed that the disabling element comprises at least one locking pin. Preferably, the locking pin implements the push button. Further preferably, the locking pin is movable from the disabling position into the enabling position and in particular vice versa, preferably by pushing the push button. It is also conceivable that the locking pin is driven, in particular by an actuator of the safety unit. In particular, in the disabling position, the locking pin mechanically blocks at least one movement of at least one component and/or element of the functional unit and/or, in the enabling position, allows and/or does not block the movement of the at least one component and/or element of the functional unit. As a result, a safety function can be implemented in a simple and/or cost-efficient way.

In addition, it is proposed that the safety unit is configured for enabling the posture function depending on at least one size adjustment, in particular a size adjustment of at least one leg unit and/or of the upper body wearing unit. For instance, the size adjustment may be an adjustment of a size of the foot unit and/or of the lower leg and/or of the upper leg and/or of the shank connection unit and/or of the seat unit and/or of the thigh connection unit. In particular, the safety unit comprises at least one size check element. Preferably, the size check element is configured for at least one inputting of at least one target size value by the user. Further preferably, the safety unit comprises at least one blocking element, which is configured for blocking the posture function if the target size differs from an actual size setting. For instance, the user may aim to set a size, for instance of the lower leg and/or of the upper leg or the like, to a certain size setting A, whereas the actual size setting is B, which is different from A. The safety unit may be configured for disabling the posture function in this case unless the actual size setting is changed to A. Preferably, the safety unit comprises at least one size comparison unit, which is configured for comparing the target size to the actual size. In particular, the size comparison unit may be implemented as a purely mechanical size comparison unit. It is also conceivable that the size comparison unit comprises at least one control unit, in particular featuring at least one processing unit, in particular electronical processing unit, which is configured for performing the size comparison. It is conceivable that the safety unit is configured for unlocking the disabling element if the target size value matches the actual size setting in such a way that the user can move the locking pin into the enabling position. It is also conceivable that the safety unit is configured for moving, in particular automatically moving, the disabling element into the enabling position if the target size value matches the actual size setting, wherein the safety unit may comprise at least one actuator for driving the disabling element. It is further conceivable that at least one target size setting is stored in the data base and/or in an internal storage element of the safety unit and assigned to the user, wherein the safety unit is preferably configured for enabling the posture function only if an actual size setting matches stored target size values which are stored for the user. As a result, accidents and/or injuries due to misadjusted components can be avoided.

Furthermore, it is proposed that the safety unit is configured for enabling the posture function depending on at least one upper leg size adjustment and/or depending on at least one lower leg size adjustment. Preferably, the safety unit and/or the size comparison unit is configured for enabling the posture function only if both the upper leg size adjustment and the lower leg size adjustment are correct, in particular match target size values for the lower leg size and the upper leg size. Preferably, the safety unit comprises at least one target size input element, in particular a dial and/or a rotating disc or the like, for inputting the upper leg target size. Further preferably, the safety unit comprises at least one, in particular additional, target size input element, in particular a dial and/or a rotating disc or the like, for inputting the lower leg target size. It is conceivable that at least one the target size input elements is implemented as an electronical input element, for instance comprising a number of buttons assigned to certain size settings, a keypad, a touch display, a control knob or the like. As a result, a correct length adjustment of a leg unit can be easily verified prior to usage of a wearable sitting posture assisting device.

In an advantageous embodiment of the invention it is proposed that the safety unit comprises at least one position detection unit, which is configured for detecting at least one relative position of at least one element with respect to at least one other element. For instance, the position detection unit may be configured for detecting a relative position of the foot connector and/or of the shank connector with respect to the knee joint, in particular along the lower leg longitudinal axis, and/or a relative position of the seat unit with respect to the knee joint, in particular along the upper leg longitudinal axis. Preferably, the position detection unit comprises at least one position detection sensor, in particular at least one linear sensor. Further preferably, the position detection unit is configured for detecting at least one actual size setting. It is conceivable that the safety unit comprises a control unit, which is configured for performing at least one comparison routine for comparing at least one sensor signal of the position detection unit to at least one target size setting, which is for instance set by the user, preferably using the target size input element. It is also conceivable that the position detection unit is implemented as a mechanical unit. In particular, the position detection unit may be mechanically coupled to the target size input element and/or to the disabling element, for instance via at least one Bowden cable and/or via at least one gear rack and/or via at least one belt element and/or via at least one gear and/or friction wheel or the like. The position detection unit may also be configured for capturing the sitting angle. In particular, it is in this case conceivable that the user has to check a functioning of the knee joint and/or of the blocking element of the knee joint prior to enabling the posture function. The position detection unit may then be configured for detecting a bending of the knee joint for the purpose of testing its functioning. As a result, a high degree of comfort and/or reliability of a safety check step can be achieved. Furthermore, a proper adjustment of a wearable sitting posture assisting device can be automatically verified during a safety check.

In another embodiment of the invention it is proposed that the safety unit is implemented as a mechanical unit, in particular an entirely mechanical unit. In particular, the safety element and/or the disabling element and/or the target size input element and/or the size comparison unit and/or the position detection unit may be implemented as a mechanical unit, in particular as an entirely mechanical unit, and an operative connection between at least some of those may in particular be implemented mechanically, in particular entirely mechanically. As a result, a high degree of cost efficiency can be achieved. Furthermore, a wearable sitting posture assisting device which is independent of a power source can be provided, in particular avoiding down times due to charging.

In a further embodiment of the invention it is proposed that the safety unit is configured for at least partly blocking the knee joint in the disabling state. Preferably, the safety unit blocks the knee joint in the disabling state, further preferably in such a manner that the knee joint cannot be bent to a lower sitting angle and/or to a higher sitting angle. In particular, the disabling element blocks the knee joint in the disabling state. Preferably, the disabling element, in particular the locking pin, connects the lower leg, in particular the lower leg support, and the upper leg, in particular the upper leg support, in the disabling state in such a way that the knee joint is blocked. As a result, a user is effectively prevented from using a wearable sitting posture assisting device in an unsafe condition.

In yet a further embodiment of the invention it is proposed that the safety unit is configured for restricting an adjustment of a sitting angle of the knee joint in the disabling state. Preferably, in the disabling state, the knee joint is in an unlocked state and in particular does not allow a person wearing the wearable sitting posture assisting device to sit down on it. In particular, the safety unit is configured for locking the actuation element of the locking unit for the knee joint. Preferably, the safety unit comprises at least one locking element for the actuation element of the locking unit for the knee joint, which preferably locks the actuation element to the upper leg in the disabling state. It is also conceivable that the safety unit is configured for lifting the restriction of the adjustment of the sitting angle automatically, for instance depending on a safety check user input regarding at least one posture function. In particular, the safety unit may comprise at least one actuator for releasing the restriction. As a result, sitting down on a wearable sitting posture assisting device is prevented unless its condition is checked for proper safety.

It is further proposed that the additional leg unit comprises at least one additional safety unit. Preferably, the safety unit and the additional safety unit are implemented at least substantially identically. Furthermore, it is conceivable that the upper body wearing unit comprises a further safety unit, which may be implemented differently from the safety unit and/or the additional safety unit, and which may further be configured for implementing at least one safety check function for the upper body wearing unit. "At least substantially identical" objects are herein to be understood as objects that are constructed and/or built in such a way that each of them can perform a certain common function. Preferably, apart from manufacturing tolerances, the objects differ only by certain elements which are irrelevant for the common function. Further preferably, apart from manufacturing tolerances, the objects are identical, wherein "identical" may encompass symmetrical with respect to each other.

Furthermore, the invention relates to a method for using a wearable sitting posture assisting device comprising a functional unit, which is configured for implementing at least one posture function, in particular a posture assisting function, for a person in at least one wearing state.

It is proposed that the method comprises at least one safety check step, in which the posture function is at least partly enabled.

By means of the invention a wearable sitting posture assisting device can be provided featuring improved characteristics regarding safety. In particular, accidents and/or injuries due to user errors can be prevented. Furthermore, safety can be improved by inducing a user to check the condition of a wearable sitting posture assisting device prior to using it. Advantageously, proper adjustment of adjustable components can be assured, in particular if several people are sharing a wearable sitting posture assisting device. In addition, low costs due to reduced maintenance effort can be achieved. Furthermore, broken components and/or imminent defects can be identified ahead of time.

Preferably, the posture function is enabled in the safety check step, in particular by the user. It is conceivable that the method comprises several safety check steps for checking the safety and/or the condition and/or the functioning of different components and/or elements and/or functions of the wearable sitting posture assisting device, in particular of the functional unit. Preferably, the user performs several safety check steps, in particular consecutively. Further preferably, the user enables at least one posture function per safety check step. For instance, the user may check a proper size adjustment of the leg unit prior to unlocking the knee joint and may check the locking unit for the knee joint prior to unlocking an adjustability of the sitting angle or the like. Of course, in an analogous manner additional safety check steps and/or additional safety check elements for other posture functions are conceivable.

Herein, the wearable sitting posture assisting device and the method according to the invention are not to be limited to the application and implementation described above. In particular, for the purpose of fulfilling a functionality herein described, the wearable sitting posture assisting device and the method according to the invention may comprise a number of respective elements, structural components, units and/or steps that differ from the number mentioned herein. Furthermore, regarding the value ranges mentioned in this disclosure, values within the limits mentioned are to be understood to be also disclosed and to be used as applicable. Furthermore, regarding the value ranges mentioned in this disclosure, values within the limits mentioned are to be understood to be also disclosed and to be used as applicable.

### Drawings

Further advantages may become apparent from the following description of the drawings. In the drawings exemplary embodiments of the invention are shown. The drawings, the description and the claims contain a plurality of features in combination. The person having ordinary skill in the art will purposefully also consider the features separately and will find further expedient combinations.

If there is more than one specimen of a certain object, in some instance only one of these may be given a reference numeral in the figures and in the description. The description of this specimen may be correspondingly transferred to the other specimens of the object.

It is shown in:
- Fig. 1: a person wearing a wearable sitting posture assisting device, in a schematic lateral view,
- Fig. 2: the person wearing the wearable sitting posture assisting device, in a schematic front view,
- Fig. 3: the person wearing the wearable sitting posture assisting device, in a schematic rear view,
- Fig. 4: a leg unit of the wearable sitting posture assisting device, in a schematic side view,
- Fig. 5: a portion of the leg unit, in a perspective view,
- Fig. 6: a schematic flow chart of a method for using the wearable sitting posture assisting device,
- Fig. 7: a portion of a leg unit of a first alternative wearable sitting posture assisting device, in a schematic side view,
- Fig. 8: a portion of the leg unit of the first alternative wearable sitting posture assisting device, in a schematic sectional front view, and
- Fig. 9: a portion of a safety unit of a second alternative wearable sitting posture assisting device, in a schematic sectional view.

### Description of the Exemplary Embodiments

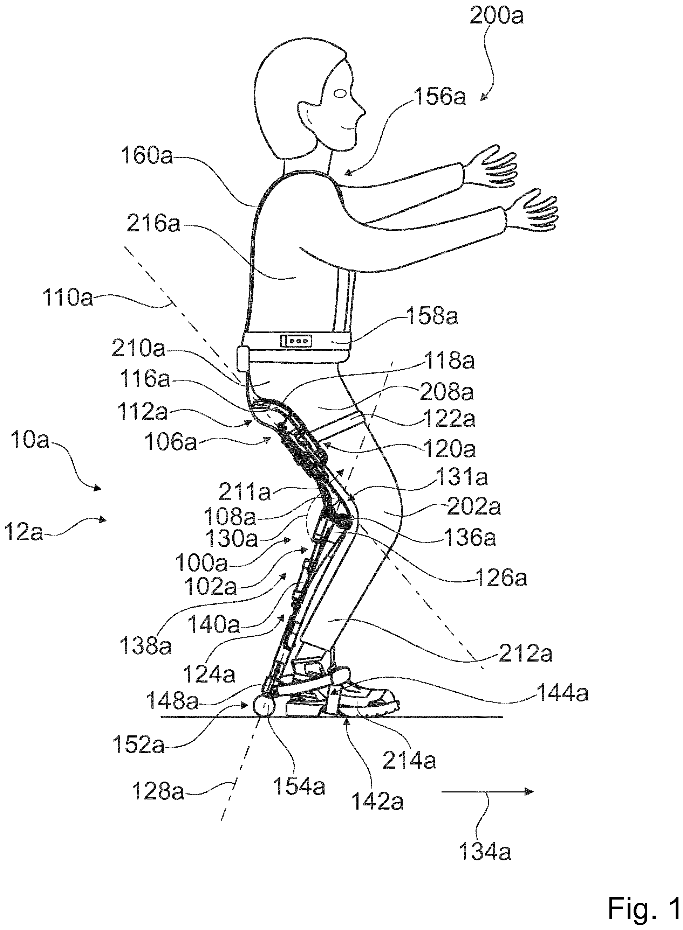

Fig. 1 shows a person 200a wearing a wearable sitting posture assisting device 100a. The wearable sitting posture assisting device 100a is configured for receiving a weight force of the person 200a in a sitting posture or in a partly sitting posture. In fig. 1 the person 200a is shown in a partly sitting posture. In the partly sitting posture a knee 202a of the person 200a is partly bent. In a sitting posture the knee 202a is bent more strongly than in the partly sitting posture. The wearable sitting posture assisting device 100a is configured for allowing the person 200a to sit down on it in different sitting postures and in different partly sitting postures. Furthermore, the wearable sitting posture assisting device 100a is configured for allowing the person 200a to walk while wearing the wearable sitting posture assisting device 100a. In addition, the wearable sitting posture assisting device 100a is configured for allowing the person 200a to stand and/or stand up and/or sit down and/or walk while wearing the wearable sitting posture assisting device 100a.

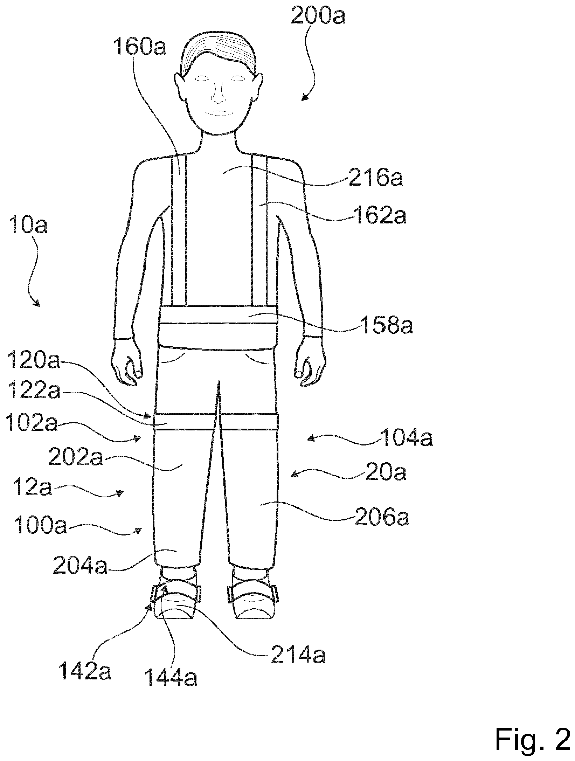

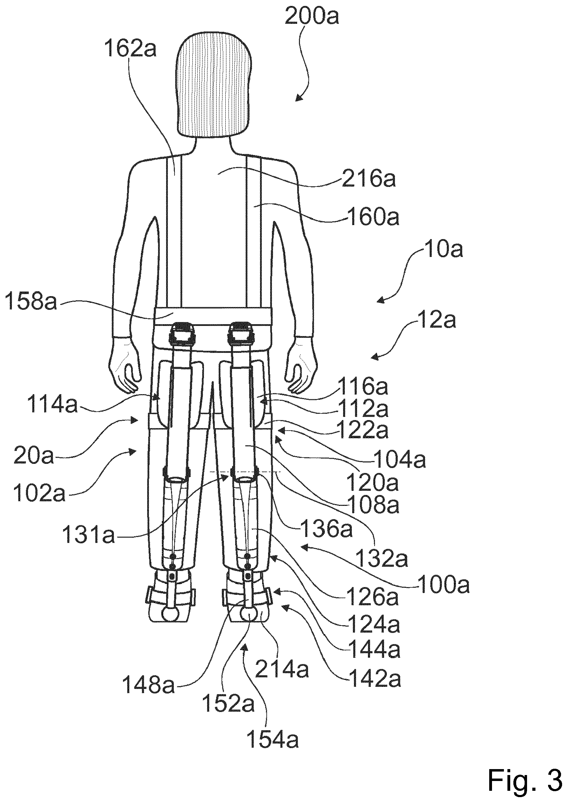

Fig. 2 shows the person 200a wearing the wearable sitting posture assisting device 100a, in a schematic front view. Fig. 3 shows the person 200a wearing the wearable sitting posture assisting device 100a, in a schematic rear view. In figs. 1 to 3 the wearable sitting posture assisting device 100a is shown in a normal wearing state. The normal wearing state encompasses a condition in which the person 200a sits or partly sits on the wearable sitting posture assisting device 100a, a condition in which the person 200a is standing up, a condition in which the person 200a is sitting down, a condition in which the person 200a stands, and a condition in which the person 200a walks, in each case while wearing the wearable sitting posture assisting device 100a. In the case shown the person 200a is wearing the wearable sitting posture assisting device 100a in a factory building, in particular while working at an assembly line. In a similar fashion it is conceivable that the person 200a wears the wearable sitting posture assisting device 100a in an office building, in a factory building, in a service building, outdoors, at work, at home, while working, during breaks, etc. Advantageously, the person 200a wears the wearable sitting posture assisting device 100a during an activity which requires the person 200a to repeatedly sit down and/or partly sit down and/or stand up and/or stand and/or walk. The person 200a can then sit down on the wearable sitting posture assisting device 100a when required, stand up while wearing the wearable sitting posture assisting device 100a when required, and walk while wearing the wearable sitting posture assisting device 100a when required.

The wearable sitting posture assisting device 100a comprises a leg unit 102a. Furthermore, the wearable sitting posture assisting device 100a comprises an additional leg unit 104a. The additional leg unit 104a is implemented identically and/or analogously to the leg unit 102a. Therefore, in the following only the leg unit 102a is described in detail. The description of the leg unit 102a is to be understood to be transferable to the additional leg unit 104a. It is also conceivable that an additional leg unit is implemented mirror-symmetrically to the leg unit 102a. In particular, it is conceivable that a leg unit and an additional leg unit are implemented as a right leg unit and a left leg unit, respectively, or vice versa.

In the case shown, the person 200a wears the leg unit 102a on a right leg 204a. The leg unit 102a is arranged on a rear side 211 a of the leg 204a of the person 200a. Furthermore, the person 200a wears the additional leg unit 104a on a left leg 206a. It is also conceivable that a person wears a leg unit on a left leg and an additional leg unit on a right leg. Furthermore, it is conceivable that a person only wears one leg unit. In addition, it is conceivable that a wearable sitting posture assisting device comprises only one leg unit. It is also conceivable that a leg unit is arranged on a lateral side of a leg and/or on a front side of a leg and/or between two legs of a person.

The person 200a sits or partly sits on the wearable sitting posture assisting device 100a in a sitting direction 134a. The person 200a faces and/or looks in the sitting direction 134a when facing forward. The sitting direction 134a is oriented parallel to a ground on which the person 200a is sitting or walking or standing.

The leg unit 102a comprises an upper leg 106a. The upper leg 106a comprises an upper leg support 108a. The upper leg 106a has an upper leg longitudinal axis 110a. The upper leg longitudinal axis 110a is oriented parallel to a plane defined by the sitting direction 134a and a surface normal of a ground the wearable sitting posture assisting device 100a stands on in the normal wearing state. The upper leg support 108a has a main extension direction which is oriented parallel to the upper leg longitudinal axis 110a. The upper leg longitudinal axis 110a is oriented parallel to a main extension direction of a thigh 208a of the leg 204a of the person, in particular when the person 200a sits, and/or partly sits and/or walks and/or stands up and/or stands while wearing the wearable sitting posture assisting device 100a.

The wearable sitting posture assisting device 100a comprises a seat unit 112a. The seat unit 112a is connected to the upper leg support 108a. In the partly sitting posture and/or in the sitting posture the person 200a sits on the seat unit 112a. In the case shown the person 200a sits on the seat unit 112a and on a seat unit 114a of the additional leg unit 104a in the partly sitting posture. The seat unit 112a comprises a sitting element 116a. The sitting element 116a contacts the thigh 208a of the person 200a. Furthermore, in the sitting posture and/or in the partly sitting posture the sitting element 116a contacts a buttock 210a of the person 200a. The seat unit 112a comprises a sitting surface 118a. The sitting element 116a comprises the sitting surface 118a. The sitting surface 118a is configured for allowing the person 200a to sit down on it with his thigh 208a and/or with his buttock 210a.

It is also conceivable that a wearable sitting posture assisting device comprises only one seat unit, in particular a common seat unit of two leg units. In particular, in this case it is conceivable that the seat unit is saddle-shaped and/or implemented in the manner of a saddle, in particular arranged between the legs of a person.

The wearable sitting posture assisting device 100a comprises a lower leg 124a. The lower leg 124a comprises a lower leg support 126a. The lower leg 124a has a lower leg longitudinal axis 128a. The lower leg longitudinal axis 128a is oriented perpendicularly to the sitting direction 134a parallel to a plane defined by the sitting direction 134a and a surface normal of a ground the wearable sitting posture assisting device 100a is standing on in the normal wearing condition. The lower leg longitudinal axis 128a and the upper leg longitudinal axis 110a are arranged in a common plane. The lower leg support 126a has a main extension direction which is oriented parallel to the lower leg longitudinal axis 128a. The lower leg longitudinal axis 128a is oriented parallel to a main extension direction of a shank 212a of the leg 204a of the person 200a, in particular when the person 200a sits, and/or partly sits and/or sits and/or stands while wearing the wearable sitting posture assisting device 100a.

The upper leg 106a and the lower leg 124a define a sitting angle 130a. The sitting angle 130a is an angle included by the upper leg longitudinal axis 110a and the lower leg longitudinal axis 128a. The sitting angle 130a is similar or identical to an angle between the thigh 208a and the shank 212a of the person 200a. The sitting angle 130a having a value between 60° and 130°, in particular a value of approximately 90°, corresponds to different sitting postures or to at least one sitting posture. The sitting angle 130a having a value between 130° and 170° corresponds to different partly sitting postures. If the person 200a is standing while wearing the wearable sitting posture assisting device 100a, the sitting angle 130a has a value between 160° and 180°, in particular a value of approximately 180°. If the person 200a is walking while wearing the wearable sitting posture assisting device 100a, the sitting angle 130a may significantly differ from 180°, in particular if the person 200a bends his knee 202a. In the sitting posture and/or in the partly sitting posture and/or when standing, the sitting angle 130a and an analogously defined additional sitting angle of the additional leg unit 104a are advantageously identical. However, it is also conceivable that the person 200a may sit on the wearable sitting posture assisting device 100a in a sitting posture or a partly sitting posture with the sitting angle 130a and the additional sitting angle differing, in particular by up to 5°, by up to 10°, by up to 15°, by up to 20°, by up to 30°, by up to 40°, or more. When the person 200a is walking while wearing the wearable sitting posture assisting device 100a, the sitting angle 130a and the additional sitting angle may significantly differ, for instance if the person 200a bends one of his knees 202a more than his other knee 202a.

The leg unit 102a comprises a knee joint 131 a, which pivotably connects the upper leg 106a to the lower leg 124a. The knee joint 131 a connects the upper leg 106a to the lower leg 124a pivotably about a knee joint axis 132a. The knee joint axis 132a is oriented perpendicularly with respect to the upper leg longitudinal axis 110a. The knee joint axis 132a is oriented perpendicularly to the lower leg longitudinal axis 128a. The knee joint axis 132a is oriented perpendicularly to the sitting direction 134a. The knee joint 131 a is partly implemented integrally with the upper leg support 108a. The knee joint 131 a is partly implemented integrally with the lower leg support 126a. The knee joint 131 a comprises at least one bearing 136a, which connects the upper leg support 108a to the lower leg support 126a.

The leg unit 102a comprises a locking unit 138a, which is configured for locking the knee joint 131 a. The locking unit 138a is configured for limiting the sitting angle 130a to a minimum value. The locking unit 138a is configured for allowing the person 200a to choose the minimum value of the sitting angle 130a. If the locking unit 138a is in a locked state, the person 200a can sit down on the wearable sitting posture assisting device 100a with the sitting angle 130a at its minimum value. The locking unit 138a is configured for being actuated by the person 200a. The locking unit 138a comprises a blocking element 140a. The blocking element 140a is a spring, in particular a gas spring. The blocking element 140a is configured for being lockable at different lengths. The blocking element 140a is connected to the upper leg support 108a. The blocking element 140a is connected to the lower leg support 126a. The blocking element 140a is configured for damping a movement of the upper leg 106a with respect to the lower leg 124a, in particular when the person 200a is sitting down.

The wearable sitting posture assisting device 100a comprises a foot unit 142a. The foot unit 142a is configured for connecting to a foot and/or shoe 214a of the person 200a. The foot unit 142a comprises a foot connector 144a for connecting to the foot and/or shoe 214a of the person 200a. The foot unit 142a comprises a foot unit support 148a. The foot unit support 148a is connected to the lower leg 124a. The foot unit support 148a is connected to the lower leg support 126a. The foot connector 144a is connected to the foot unit support 148a.

The wearable sitting posture assisting device 100a comprises a thigh connection unit 120a for connecting to the thigh 208a of the person 200a. The thigh connection unit 120a is connected to the upper leg support 108a. The thigh connection unit 120a is configured for connecting the upper leg 106a to the thigh 208a of the person 200a. The thigh connection unit 120a comprises a thigh strap 122a. The thigh strap 122a is fixed to the thigh 208a of the person 200a.

The leg unit 102a comprises a ground contact unit 152a. The ground contact unit 152a is connected to the lower leg support 126a. The ground contact unit 152a comprises a ground contact element 154a. When the person 200a sits or partly sits on the wearable sitting posture assisting device 100a, the ground contact unit 152a, in particular the ground contact element 154a, is in contact with the ground. The ground contact unit 152a, in particular the ground contact element 154a, is configured for transmitting a portion of a weight force of the person 200a to the ground. The ground contact element 154a is made of rubber. However, other shapes and/or materials are conceivable for a ground contact element as mentioned above.

When the person 200a sits or partly sits on the wearable sitting posture assisting device 100a, the weight force of the person 200a is at least partly, in particular directly or indirectly, transmitted from the seat unit 112a to the upper leg support 108a; from the upper leg support 108a to the knee joint 131 a; from the knee joint 131 a to the lower leg support 126a; from the lower leg support 126a to the ground contact element 154a; and from the ground contact element 154a to the ground.

In particular, the weight force of the person 200a is additionally transmitted to the ground via a foot and/or shoe 214a of the person 200a. Preferably, the ground contact element 154a is arranged on a rear side of the foot and/or shoe 214a of the person 200a. When the person 200a sits or partly sits on the wearable sitting posture assisting device 100a, the foot and/or shoe 214a of the person 200a is in contact with the ground in addition to the ground contact element 154a. Preferably, the ground contact element 154a is arranged contactlessly with respect to the ground when the person 200a walks and/or stands while wearing the wearable sitting posture assisting device 100a.

The wearable sitting posture assisting device 100a comprises an upper body wearing unit 156a. The person 200a wears the upper body wearing unit 156a on his upper body 216a, which upper body 216a may include hips and/or a waist of the person 200a. The upper body wearing unit 156a comprises a belt 158a. Furthermore, the upper body wearing unit 156a comprises suspenders 160a, 162a. The leg unit 102a is connected to the upper body wearing unit 156a. The additional leg unit 104a is connected to the upper body wearing unit 156a. It is conceivable that an upper body wearing unit comprises no belt and only suspenders, or vice versa. It is also conceivable that a wearable sitting posture assisting device is only connected to legs and/or feet and/or shoes of a person who it is worn by. Furthermore, it is conceivable that a wearable sitting posture assisting device comprises no upper body wearing unit.

The wearable sitting posture assisting device 100a comprises at least one functional unit 10a configured for implementing, in the wearing state, at least one posture function, in particular at least one posture assisting function, for the person 200a. In the case shown the functional unit 10a comprises the leg unit 102a. Furthermore, the functional unit 10a comprises the additional leg unit 104a. In addition, the functional unit 10a comprises the upper body wearing unit 156a. The posture function may encompass an adjustment of the sitting angle 130a, a size adjust of the leg unit 102a and/or of the additional leg unit 104a, in particular of the lower leg 124a and/or of the upper leg 106a and/or of the seat unit 112a, blocking the knee joint 131 a at a certain sitting angle 130a when sitting down, standing up while wearing the wearable sitting posture assisting device 100a or the like.

Furthermore, the wearable sitting posture assisting device 100a comprises at least one safety unit 12a, which at least partly disables the posture function in at least on disabling state and which at least partly enables the posture function in at least one enabling state. In the embodiment shown the safety unit 12a disables the posture function in the disabling state. Furthermore, the safety unit 12a enables the posture function in the enabling state. In particular, the safety unit 12a does not disable the posture function in the enabling state. In the case shown, the leg unit 102a comprises the safety unit 12a. Furthermore, the additional leg unit 104a comprises an additional safety unit 20a. In the case shown, the safety unit 12a and the additional safety unit 20a are implemented identically. Therefore, in the following only the safety unit 12a is described in detail. However, it is conceivable that the safety unit 12a and the additional safety unit 20a are implemented differently, for instance regarding a handedness, in particular if the leg unit 102a is a right leg unit and the additional leg unit 104a is a left leg unit or vice versa. Furthermore, it is conceivable that the functional unit 10a comprises a common safety unit for the leg unit 102a and the additional leg unit 104a.

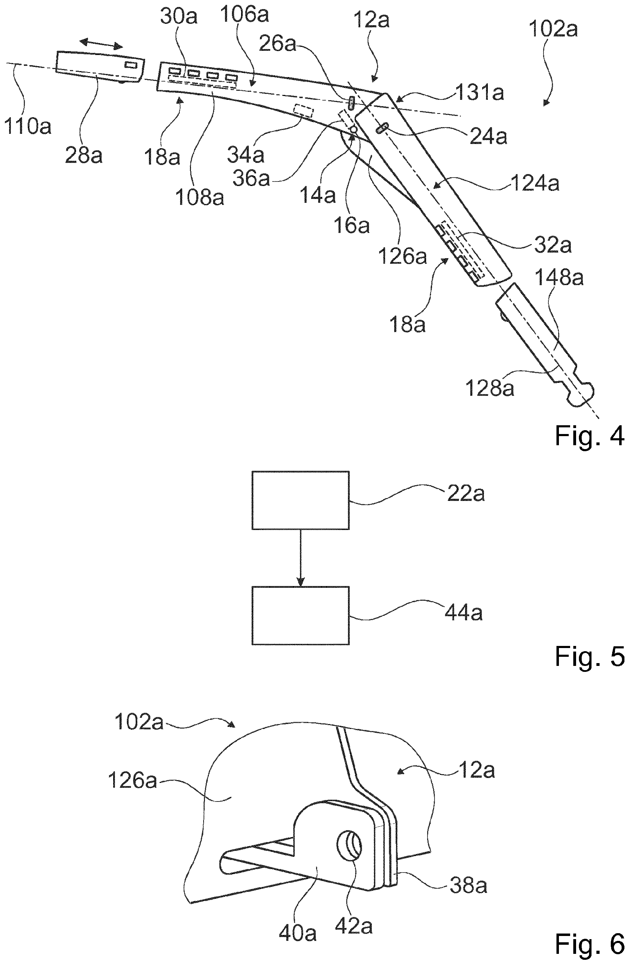

Figure 4 shows the leg unit 102a in a schematic side view. For reasons of clarity some elements and/or units of the leg unit 102a are omitted in figure 4. The safety unit 12a blocks at least one movement in the disabling state. In the case shown, the safety unit 12a blocks a movement of the upper leg 106a with respect to the lower leg 124a, in particular a bending and/or stretching of the knee joint 131 a. The safety unit 12a is configured for at least partly blocking the knee joint 131 a in the disabling state.

The safety unit 12a comprises at least one disabling element 14a which is, in the disabling state, in at least one disabling position and which is, in the enabling state, in at least one enabling position. The disabling element 14a is movable in a direction parallel to the knee joint axis 132a and/or along the knee joint axis 132a (compare figure 3). In the disabling position the disabling element 14a connects the upper leg support 108a and the lower leg support 126a, in particular in such a way that they are not movable with respect to each other around the knee joint axis 132a. In the case shown, the disabling element 14a is in the enabling position located entirely inside the lower leg support 126a. In particular, the upper leg support 108a is movable past the disabling element 14a in the enabling state. Furthermore, the disabling element 14a is in the disabling position partly located inside the upper leg support 108a and partly located inside the lower leg support 126a, in particular connecting the upper leg support 108a and the lower leg support 126a in a twist-proof manner.

In the case shown, the disabling element 14a comprises at least one locking pin 16a. It is conceivable that the disabling element 14a is implemented as the locking pin 16a. In the case shown, the locking pin 16a is implemented as a metal pin, however other materials are conceivable. Additionally or alternatively, the disabling element 14a may comprise at least one claw element and/or at least one form-locking element and/or at least one friction element or may be implemented as such.

The safety unit 12a is configured for enabling the posture function depending on at least one user input. The user input may comprise at least one mechanical input, for instance at least one unlocking and/or at least one pulling and/or at least one pushing and/or at least one turning or the like. In particular, the user checks a certain function and/or component and/or adjustment or the like of the wearable sitting posture assisting device, in particular a safety thereof, and generates the user input if safety has been checked successfully. The safety unit 12a thus enables the posture function only if the wearable sitting posture assisting device is in a proper working condition. As mentioned above, the safety unit 12a may comprise at least one, in particular electronical, input interface, for instance a touch display, at least one button, a fingerprint scanner, a voice recognition unit, an iris scanner or the like.

The safety unit 12a is configured for enabling the posture function depending on at least one size adjustment. In particular, the safety unit 12a is configured for comparing at least one target size value, which is in particular input by the user, to at least one actual size setting value. The size adjustment may for instance comprise a size adjustment of the leg unit 102a and/or of the seat unit 112a and/or of the upper body wearing unit 156a.

In the case shown, the safety unit 12a is configured for enabling the posture function depending on at least one upper leg size adjustment and/or depending on at least one lower leg size adjustment. In particular, the safety unit 12a comprises a first target size input element 24a. The first target size input element 24a is configured for inputting a target size of the lower leg 124a, in particular of a lower leg length. For instance, the foot unit support 148a may be movable along the lower leg longitudinal axis 128a, preferably into different positions, for instance corresponding to a small, medium, large and extra large size setting. The first target size input element 24a may then be configured for inputting these possible size setting values as target size values. For instance, the first target size input element 24a may comprise at least one dial and/or at least one rotating disc and/or at least one slider and/or at least one knob element or the like for inputting the target size value.

Furthermore, the safety unit 12a comprises a second target size input element 26a. The second target size input element 26a is configured for inputting a target size value of the upper leg 106a, in particular of an upper leg length. For instance, the upper leg 106a may comprise a support element 28a, which is movable along the upper leg longitudinal axis 110a for the purpose of a length adjustment, in particular analogously to the foot unit support 148a in case of the lower leg 124a. In the case shown, the support element 28a is connected to the seat unit 112a and in particular allows adjusting a position of the seat unit 112a with respect to the knee joint 131 a, preferably along the upper leg longitudinal axis 110a. For instance, the second target size input element 26a may comprise at least one dial and/or at least one rotating disc and/or at least one slider and/or at least one knob element or the like for inputting the target size value.

The safety unit 12a comprises at least one position detection unit 18a, which is configured for detecting at least one relative position of at least one element with respect to at least one other element. In the case shown, the position detection unit 18a is configured for detecting a position of the support element 28a with respect to the upper leg support 108a. Furthermore, the position detection unit 18a is configured for detecting a position of the foot unit support 148a with respect to the lower leg support 126a. In the case shown, the position detection unit 18a comprises a first sensor 30a, in particular a linear sensor, and comprises a second sensor 32a, in particular a linear sensor, for detecting these positions.

The safety unit 12a comprises at least one control unit 34a. The control unit 34a is connected to the position detection unit 18a, in particular to the first sensor 30a and to the second sensor 32a. Furthermore, the control unit 34a is connected to the first target size input element 24a and to the second target size input element 26a. In addition, the control unit 34a is connected to an actuator 36a for the disabling element 14a, which is configured for moving the disabling element 14a from the disabling position into the enabling position and back, in particular upon receiving at least one control signal from the control unit 34a. The control unit 34a is configured for determining at least one actual size setting value depending on at least one signal received from the position detection unit 18a. Furthermore, the control unit 34a is configured for comparing the actual size setting value to at least one target size value which has been inputted via the first target size input element 24a and/or via the second target size input element 26a. The control unit 34a implements a size comparison unit. In the case shown, the control unit 34a triggers a movement of the disabling element 14a from the disabling position into the enabling position only if an upper leg target size value matches an actual upper leg size setting and a lower leg target size value matches an actual lower leg size setting. Additionally or alternatively to the target size input elements 24a, 26a, it is conceivable that the safety unit 12a comprises at least one user detection unit for identifying a user, in particular based on at least one user data set stored in a data base to which the safety unit 12a may be connectable, and/or in an internal storage. Target size values for the user may then be acquired from the user data set and compared to actual size setting values during a safety check.

It is also conceivable that the safety unit 12a is implemented entirely as a mechanical unit. In particular, the position detection unit 18a, the disabling element 14a and the target size input elements 24a, 26a may be implemented entirely as mechanical units respectively elements. For instance, the safety unit 12a may comprise at least one lock element which blocks a movement of the disabling element 14a. Such a lock element may be mechanically unlocked if target size values match actual size setting values. The user may then be enabled to manually move the disabling element 14a from the disabling position into the enabling position in order to enable the posture function.

Figure 5 shows a portion of the leg unit 102a in a perspective view. The safety unit 12a is configured for restricting an adjustment of the sitting angle 130a of the knee joint 131 a in the disabling state. The safety unit 12a comprises an additional disabling element 38a. In the case shown, the additional disabling element 38a is permanently connected to the lower leg support 126a. The additional disabling element 38a is arranged next to an actuation element 40a of the locking unit 138a for the knee joint 131 a. The actuation element 40a is configured for enabling a sitting angle adjusting function of the locking unit 138a. In particular, the actuation element 40a is implemented as a lever, which is to be pulled or pushed by the user in order to adjust the sitting angle 130a. In the disabling state the actuation element 40a is fixed to the additional disabling element 38a by means of at least one fixing element, which is not shown in figure 5. The fixing element may for instance be implemented as a locking pin, a lock, a locking ring, a combination lock, a padlock or the like. In the case shown, the actuation element 40a and the additional disabling element 38a implement a through-hole 42a for the fixing element, in which through-hole 42a the fixing element is at least partly located in the disabling state. Preferably, the knee joint 131 a is, in the disabling state, in an unlocked state. Further preferably, the user has to unlock the actuation element 40a from the disabling element 38a in order to enable a sitting angle adjustment function and/or in order to enable a sitting function of the wearable sitting posture assisting device 100a.

Figure 6 shows a schematic flow chart of a method for using the wearable sitting posture assisting device 100a. The method comprises at least one safety check step 22a. At least one posture function is at least partly enabled in the safety check step 22a prior to wearing the wearable sitting posture assisting device 100a. In the case shown, a proper size adjustment of the leg unit 102a is checked in the safety check step 22a. The knee joint 131 a is unlocked in the safety check step 22a, in particular by moving the disabling element 14a from the disabling position into the enabling position (compare figure 4). Furthermore, a visual inspection of the leg unit 102a is performed in an additional safety check step 44a, for instance in order to determine whether there is a need for maintenance. The actuation element 40a (compare figure 5) is unlocked in the additional safety check step 44a. Preferably, the safety check step 22a and/or the additional safety check step 44a are performed by the user prior to using the wearable sitting posture assisting device 100a.

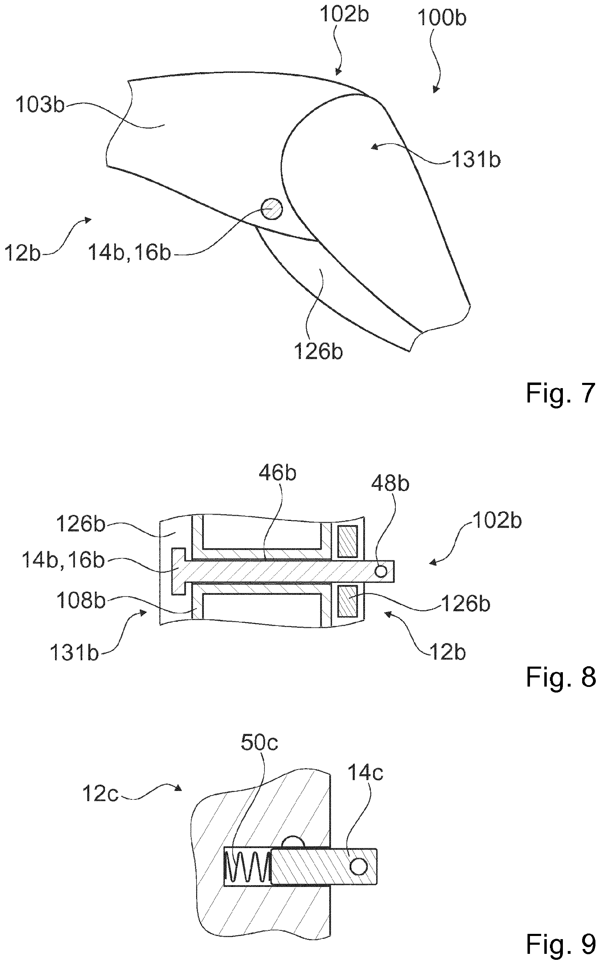

Figs. 7 to 9 show further exemplary embodiments of the invention. The following description is substantially limited to the differences between the exemplary embodiments, wherein regarding structural elements, features and functions that remain the same the description of the other exemplary embodiments, in particular the exemplary embodiment of figs. 1 to 6, may be referred to. For distinguishing the exemplary embodiments, the letter a of the reference numerals in the exemplary embodiment of figs. 1 to 6 has been substituted by the letters b and c in the reference numerals of the exemplary embodiments of figs. 7 to 9. Regarding structural elements having the same denomination, in particular regarding structural elements having the same reference numerals, principally the drawing and/or the description of the other exemplary embodiments, in particular of the exemplary embodiment of figs. 1 to 6, may be referred to.

Figure 7 shows a portion of a leg unit 102b of a first alternative wearable sitting posture assisting device 100b in a schematic side view. The wearable sitting posture assisting device 100b comprises a safety unit 12b, which at least partly disables at least one posture function in at least one disabling state and which at least partly enables the posture function in at least one enabling state. In the case shown, the safety unit 12b blocks in the disabling state a movement of a knee joint 131 b of the leg unit 102b.

Figure 8 shows a portion of the leg unit 102b with the knee joint 131 b in a sectional front view. The safety unit 12b comprises a disabling element 14b, which is implemented as a locking pin 16b. In the disabling state the disabling element 14b protrudes partly through an upper leg support 108b, and a lower leg support 126b of the leg unit 102b. The upper leg support 108b and the lower leg support 126b together implement a through-hole 46b for the disabling element 14b. The disabling element 14b features a hole 48b for insertion of a lock element, for instance a locking splint, a locking clip, a lock, a locking ring or the like. For enabling the posture function, the disabling element 14b is unlocked and pulled out of the through-hole 46b, preferably by a user, during a safety check step. The safety unit 12b is implemented entirely as a mechanical unit.

Figure 9 shows a portion of a safety unit 12c of a second alternative wearable sitting posture assisting device, which is not shown in the figure. The safety unit 12c comprises at least one disabling element 14c, which is, in at least one disabling state of the safety unit 12c, in at least one disabling position and which is, in at least one enabling state of the safety unit 12c, in at least one enabling position. In the case shown, the enabling position is a pushed-in position of the disabling element 14c. Furthermore, the disabling position is a pushed-out position of the disabling element 14c. The disabling element 14c is implemented as a bistable push button, wherein a first push moves the disabling element 14c from the disabling position into the enabling position and wherein a second push after the first push moves the disabling element 14c from the enabling position back into the disabling position. In the case shown, the safety unit 12c features at least one spring element 50c for pushing the disabling element 14c into the disabling position. The disabling element 14c latches in the enabling position. A latching of the disabling element 14c in the enabling position is released upon pushing the disabling element 14c deeper in. The disabling element 14c may for instance, in the disabling position, block a movement of an upper leg with respect to a lower leg.

It is conceivable that other functions of a wearable sitting posture assisting device are disabled and/or enabled by a safety unit, in particular for the purpose of implementing a safety check routine, for instance a seat unit adjustment function, a movement of a foot unit and/or a shoe connector, an adjustability of an upper body wearing unit and the like.

## Claims

1. Wearable sitting posture assisting device, comprising a functional unit (10a) which is configured for implementing at least one posture function, in particular a posture assisting function, for a person (200a) in at least one wearing state, **characterized by** at least one safety unit (12a-c) which, in at least one disabling state, at least partly disables the posture function and which, in at least one enabling state, at least partly enables the posture function.

2. Wearable sitting posture assisting device according to claim 1, **characterized in that,** in the disabling state, the safety unit (12a-c) blocks at least one movement.

3. Wearable sitting posture assisting device according to claim 1 or 2, **characterized in that** the safety unit (12a-c) is configured for enabling the posture function depending on at least one user input.

4. Wearable sitting posture assisting device according to any one of the preceding claims, **characterized in that** the safety unit (12a-c) comprises at least one disabling element (14a-c), which is, in the disabling state, in at least one disabling position and which is, in the enabling state, in at least one enabling position.

5. Wearable sitting posture assisting device according to claim 4, **characterized in that** the disabling position and/or the enabling position is a pushed-in position of the disabling element (14c).

6. Wearable sitting posture assisting device according to claim 4 or 5, **characterized in that** the disabling element (14a-c) comprises at least one locking pin (16a-c).

7. Wearable sitting posture assisting device according to any one of the preceding claims, **characterized in that** the safety unit (12a) is configured for enabling the posture function depending on at least one size adjustment.

8. Wearable sitting posture assisting device according to claim 7, **characterized in that** the safety unit (12a) is configured for enabling the posture function depending on at least one upper leg size adjustment and/or depending on at least one lower leg size adjustment.

9. Wearable sitting posture assisting device according to any one of the preceding claims, **characterized in that** the safety unit (12a) comprises at least one position detection unit (18a), which is configured for detecting at least one relative position of at least one element with respect to at least one other element.

10. Wearable sitting posture assisting device according to any one of the preceding claims, **characterized in that** the safety unit (12b; 12c) is implemented as a mechanical unit.

11. Wearable sitting posture assisting device according to any one of the preceding claims, **characterized in that** the functional unit (10a; 10b) comprises at least one leg unit (102a; 102b) featuring at least one knee joint (131 a; 131 b).

12. Wearable sitting posture assisting device according to claim 11, **characterized in that** the safety unit (12a; 12b) is configured for at least partly blocking the knee joint (131 a; 131 b) in the disabling state.

13. Wearable sitting posture assisting device according to claim 11 or 12, **characterized in that** the safety unit (12a) is configured for restricting, in the disabling state, an adjustment of a sitting angle (130a) of the knee joint (131 a).

14. Wearable sitting posture assisting device according to any one of claims 11 to 13, **characterized by** at least one additional leg unit (104a) and by at least one additional safety unit (20a).

15. Method for using a wearable sitting posture assisting device (100a), in particular according to any one of the preceding claims, comprising a functional unit (10a) configured for implementing at least one posture function, in particular a posture assisting function, for a person (200a) in at least one wearing state, **characterized by** at least one safety check step (22a), in which the posture function is at least partly enabled.
